# EUROPEAN PATENT APPLICATION

(11) **EP 4 652 886 A1**
(43) Date of publication of application: **26.11.2025**
(21) Application number: 25176662.2
(22) Date of filing: 15.05.2025
(51) Int. Cl.: A24F 40/485, A24F 40/50, A24F 40/57, A61M 11/04, A61M 15/06

(54) **VAPORIZATION APPARATUS AND VAPORIZATION DEVICE**

(30) Priority: 22.05.2024 CN 202421131008 U
(71) Applicant: Shenzhen Geekvape Technology Co., Ltd., Shenzhen, Guangdong 518100 (CN)
(72) Inventor: LI, Zhenjun, Shenzhen, 518100 (CN); CHEN, Qiang, Shenzhen, 518100 (CN)
(74) Representative: Manitz Finsterwald Patent- und Rechtsanwaltspartnerschaft mbB

(57) **Abstract**

Disclosed are a vaporization apparatus and a vaporization device. The vaporization apparatus including a housing 2, a vaporizer 6, an installation base 3, a control circuit board 4, and an airflow control assembly 5. The installation base is formed with an air intake channel 30 communicating with the air inlet end 61 of the vaporizer. The airflow control assembly comprises an adjustment member 51 and a power control switch 52, wherein the adjustment member is disposed at the air inlet port 22 and is movably connected to the housing. The adjustment member includes a shielding portion (53) and a connecting portion (54), with the connecting portion being connected to the power control switch 52. Through the adjustment member's connection with both the air intake channel and the power control switch, the adjustment member is capable of moving relative to the housing.

## Description

### TECHNICAL FIELD

The present application relates to the field of electronic vaporization technology, and specifically to a vaporization apparatus and a vaporization device.

### BACKGROUND

In electronic vaporization devices, the operational power of a vaporization core is typically adjusted based on user needs to regulate the ratio of aerosol generated by the device. However, the actual amount of aerosol discharged depends on the device's air intake volume. In conventional technologies, power adjustment and airflow adjustment are controlled separately. If the vaporization core operates at high power with insufficient airflow, aerosol cannot be fully discharged, leading to localized overheating, degraded user experience, or even device damage. Conversely, low power with excessive airflow results in overly diluted aerosol output.

### SUMMARY

The present application provides a vaporization apparatus and vaporization device to resolve the issue in existing technologies where mismatched power and airflow adjustments cause device damage and poor user experience.

According to one aspect of the application, an embodiment provides a vaporization apparatus, comprising:
A housing, wherein the housing forms an inner accommodating cavity, the inner accommodating cavity including a liquid storage chamber configured to store an aerosol-generating substrate; the housing further defining an air inlet port and an air outlet port in communication with the external environment;
A vaporizer, wherein the vaporizer is disposed within the liquid storage chamber; the vaporizer comprising an air inlet end and an air outlet end arranged oppositely, wherein the air outlet end communicates with the air outlet port;
An installation base, wherein the installation base is fixedly assembled to an end of the liquid storage chamber distal from the air outlet port, sealing the liquid storage chamber; the installation base is formed with an air intake channel communicating with the air inlet end of the vaporizer;
A control circuit board, wherein the control circuit board is electrically connected to the vaporizer and is configured to control the operation of the vaporizer;
An airflow control assembly, wherein the airflow control assembly comprises an adjustment member and a power control switch, the adjustment member is disposed at the air inlet port and is movably connected to the housing; the adjustment member includes a shielding portion covering the air intake channel, the shielding portion is provided with at least one airflow aperture communicating with the air inlet port; the adjustment member further includes a connecting portion linked to the power control switch; the power control switch is electrically connected to the control circuit board to regulate the operational power of the vaporizer; the adjustment member is movable relative to the housing to alter the total effective area of the airflow aperture communicating with the air intake channel, while simultaneously drives the power control switch to synchronously adjust the operational power of the vaporizer.

In one embodiment, the number of airflow apertures is at least two; the adjustment member is movable relative to the housing to vary the number of airflow apertures communicating with the air intake channel, thereby changing the total effective area.

In another embodiment, the number of airflow apertures is one; the adjustment member is movable relative to the housing to adjust the total effective area of the airflow aperture communicating with the air intake channel.

In a further embodiment, two sides of the adjustment member are provided with elastic protrusions, and an inner wall of the housing is provided with at least two sets of the positioning grooves matching the elastic protrusions; the elastic protrusions are movably engaged with one set of the positioning grooves, and the adjustment member is switchable under external force to engage with any set of the positioning grooves.

In another embodiment, the adjustment member comprises a movable base and a sliding button fixedly interconnected; the movable base is disposed within the inner accommodating cavity of the housing, with the shielding portion and the connecting portion both formed on the movable base; the sliding button is exposed outside the housing through the air inlet port.

In one embodiment, the sliding button and the movable base are integrally formed; or, the sliding button and the movable base are fixedly connected.

In another embodiment, the connecting portion comprises a groove, and the power control switch includes an actuating lever inserted into the groove to fixedly couple the actuating lever to the connecting portion; the movement of the adjustment member relative to the housing drives the actuating lever to move relative to the control circuit board, thereby controlling the operational power of the vaporizer.

In a further embodiment, the control circuit board is disposed between the airflow control assembly and the installation base; the installation base includes an air guide tube communicating with the air intake channel, the air guide tube penetrates the control circuit board through and is fixedly connected to the control circuit board.

In one configuration, an end of the air guide tube is provided with an elastic sealing flange elastically contacting the shielding portion to seal the air intake channel.

According to another aspect of the application, an embodiment provides a vaporization device, comprising a power supply assembly and the vaporization apparatus according to any one of claims 1-9; the power supply assembly is fixedly disposed within the inner accommodating cavity of the housing and is configured to supply power to the vaporizer.

The vaporization apparatus and vaporization device according to the preceding embodiments, through the adjustment member's connection with both the air intake channel and the power control switch, the adjustment member is capable of moving relative to the housing. By adjusting the position of the adjustment member relative to the housing, synchronized adjustment of both airflow volume and operational power of the vaporizer can be achieved by adjustment member in a single operation. This configuration thereby effectively avoids mismatching between airflow volume and operational power, extends the service life of the vaporization device, and improves user experience.

### BRIEF DESCRIPTION OF THE DRAWINGS

FIG. 1 is a schematic structural diagram of a vaporization apparatus provided by an embodiment of the present application;
FIG. 2 is a cross-sectional view of the vaporization apparatus provided by an embodiment of the present application;
FIG. 3 is a bottom view of the vaporization apparatus provided by an embodiment of the present application;
FIG. 4 is a structural diagram of an installation base provided by an embodiment of the present application;
FIG. 5 is a cross-sectional view of the installation base provided by an embodiment of the present application;
FIG. 6 is a structural diagram of an adjustment member of the vaporization apparatus provided by an embodiment of the present application;
FIG. 7 is a bottom view of the adjustment member provided by an embodiment of the present application;
FIG. 8 is a top view of the adjustment member provided by an embodiment of the present application;
FIG. 9 is a structural diagram of a power control switch provided by an embodiment of the present application;
FIG. 10 is a partial schematic diagram of a housing provided by an embodiment of the present application;
FIG. 11 is a diagram illustrating power level correspondences of the vaporization apparatus provided by an embodiment of the present application.

List of Reference Numerals:
1-vaporization apparatus;
2-housing; 20-inner accommodating cavity; 21-liquid storage chamber; 22-air inlet port; 23-air outlet port; 24-positioning grooves;
3-installation base; 30-air intake channel; 31-air guide tube; 32-elastic sealing flange;
4-control circuit board;
5-airflow control assembly; 51-adjustment member; 52-power control switch; 53-shielding portion; 530-airflow aperture; 54-connecting portion; 55-elastic protrusions; 56-movable base; 57-sliding button; 58-groove; 59-actuating lever;
6-vaporizer; 61-air inlet end; 62-air outlet end;
7-power supply assembly.

### DETAILED DESCRIPTION

The present application is further explained in detail below through specific embodiments in conjunction with the accompanying drawings. Similar components in different embodiments are labeled with related analogous reference numerals. In the following embodiments, many details are described to facilitate a better understanding of the present application. However, those skilled in the art may readily recognize that some features may be omitted under varying circumstances or substituted with other components, materials, or methods. In certain cases, some operations related to the present application are not explicitly illustrated or described in the specification. This is to avoid obscuring the core aspects of the present application with excessive descriptions. For those skilled in the art, detailed explanations of such operations are unnecessary, as they can fully comprehend the relevant operations based on the descriptions herein and general technical knowledge in the field.

Furthermore, the features, operations, or characteristics described in the specification may be combined in any suitable manner to form various embodiments. The operational steps involved in each embodiment may also be reordered or adjusted in ways that would be obvious to those skilled in the art. Therefore, the descriptions and drawings are intended to clearly illustrate specific embodiments and do not imply mandatory components, arrangements, or sequences.

In the present text, numerical designations such as "first," "second," and the like assigned to components are solely for distinguishing described objects and carry no sequential or technical implications. The terms "connected" or "coupled" as used in this application, unless otherwise specified, encompass both direct and indirect connections or couplings.

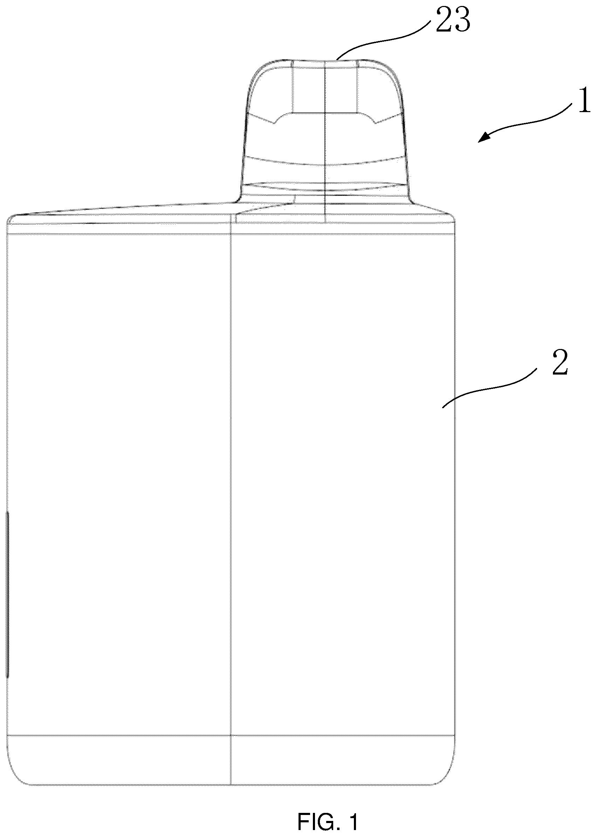

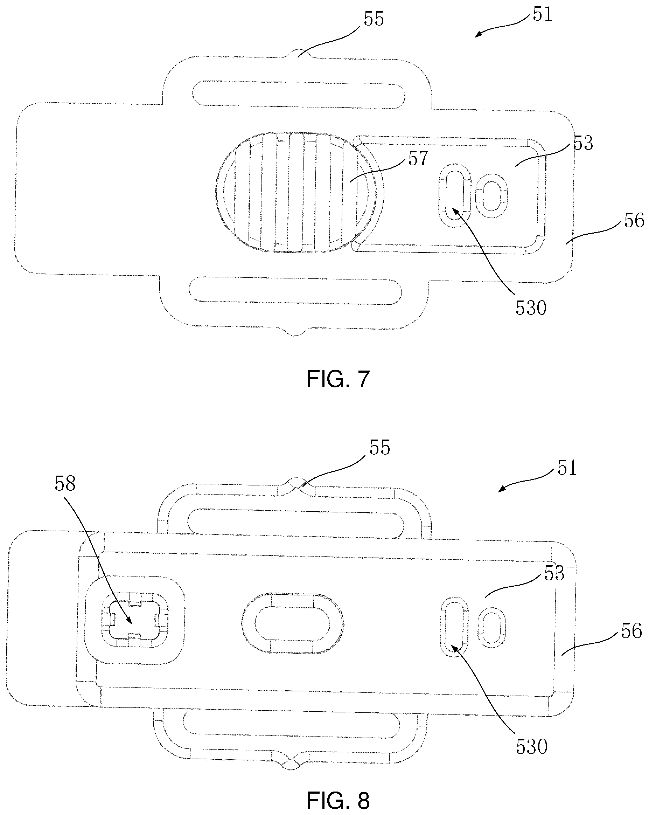

To address the issues in the prior art where separate control of power adjustment and air intake regulation in vaporization devices is prone to mismatch between power output and air intake volume, thereby resulting in susceptibility to damage of the vaporization device and suboptimal user experience, the embodiments of the present application provide a vaporization apparatus 1. As shown in FIGS. 1-10, the vaporization apparatus 1 comprises: a housing 2, a vaporizer 6, an installation base 3, a control circuit board 4, and an airflow control assembly 5.

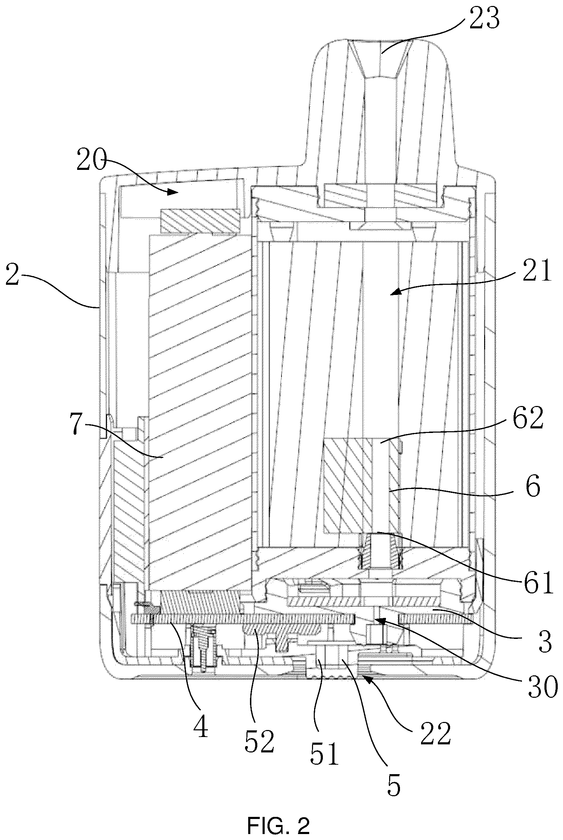

As shown in FIG. 2, the housing 2, serving as the outermost structure of the vaporization apparatus 1, defines an inner accommodating cavity 20 internally to house various components required for the operation of the vaporization apparatus 1. The inner accommodating cavity 20 may be configured as either a fully interconnected chamber or partitioned into partially enclosed subcompartments that are independent of one another. The specific configuration of these subcompartments is designed based on the functional requirements of the components housed within them and the connectivity needs between these components. For instance, portions of the cavity may be isolated to ensure structural integrity or to optimize airflow and electrical connections between critical parts.

In the embodiments of the present application, the inner accommodating cavity 20 includes a liquid storage chamber 21 configured to hold an aerosol-generating substrate. The aerosol-generating substrate within the liquid storage chamber 21 may be retained and delivered via a capillary liquid-retaining member, which includes, but is not limited to, liquid-retaining cotton or a porous ceramic structure.

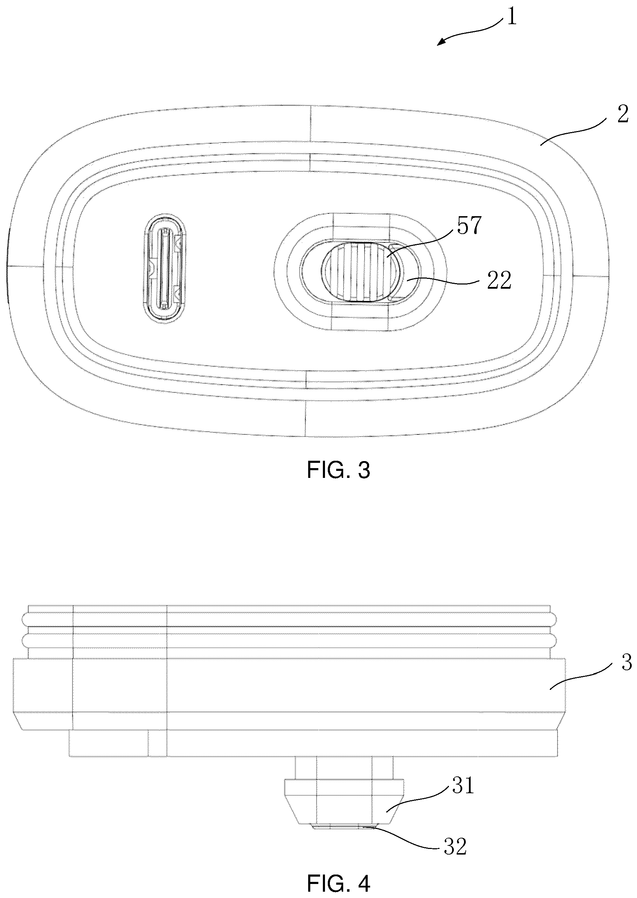

As illustrated in FIG. 3, to expel aerosol generated by heating the aerosol-generating substrate, the housing 2 is equipped with an air inlet port 22 and an air outlet port 23, both of which are in communication with the external environment. Ambient air enters the vaporization apparatus 1 through the air inlet port 22, while the aerosol produced by heating the substrate is expelled via the air outlet port 23. The embodiments of the present application do not restrict the specific positional arrangement of the air inlet port 22 and air outlet port 23 on the housing 2. Critically, as long as the airflow pathway between the air inlet port 22 and air outlet port 23 traverses the vaporizer 6, the intended functionality is achieved: air flows into the vaporization apparatus 1 through the air inlet port 22, the vaporizer 6 vaporizes the aerosol-generating substrate to generate aerosol, which mixes with the incoming air, and the resulting aerosol-air mixture is discharged through the air outlet port 23.

The vaporizer 6 is disposed within the liquid storage chamber 21. It transfers the aerosol-generating substrate in the liquid storage chamber 21 through a liquid-conducting component and heats the aerosol-generating substrate during operation to generate aerosol. The vaporizer 6 includes an air inlet end 61 and an air outlet end 62 arranged opposite to each other, with the air outlet end 62 connected to the air outlet port 23. Air enters the vaporizer 6 through the air inlet end 61. The aerosol generated by the heating of the aerosol-generating substrate by the vaporizer 6 mixes with the air, exits through the air outlet end 62 of the vaporizer 6, and is ultimately discharged outside the vaporization apparatus 1 via the air outlet port 23 of the housing 2.

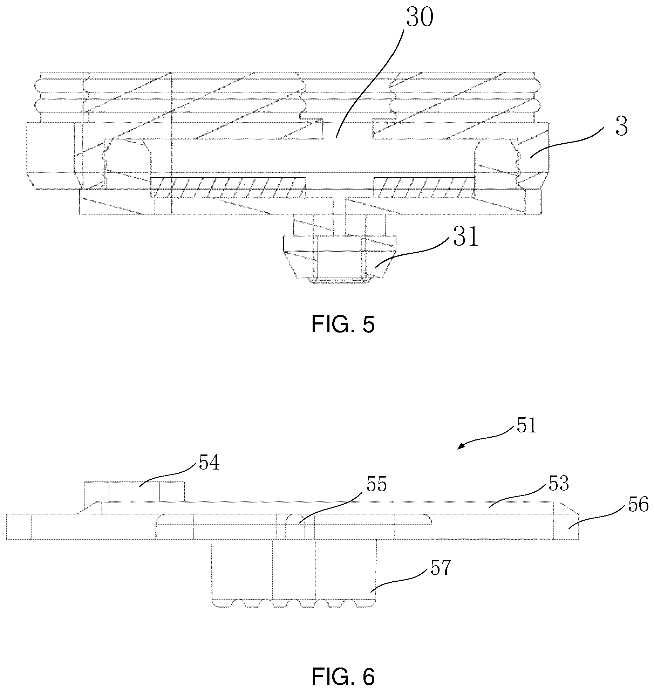

As shown in FIG. 4 and 5, the installation base 3 is fixedly assembled at the end of the liquid storage chamber 21 away from the air outlet port 23, the installation base 3 sealingly covers the liquid storage chamber 21 to enclose the liquid storage chamber 21 and form a relatively sealed chamber. To allow external airflow into the vaporizer 6, the installation base 3 is integrated with an air intake channel 30 that communicates with the air inlet end 61 of the vaporizer 6. This configuration enables air to enter the vaporizer 6 through the air intake channel 30.

In this configuration, the installation base 3 may be integrally molded with the housing 2 to enclose and define the liquid storage chamber 21. Alternatively, the installation base 3 can be premanufactured separately from the housing 2 and subsequently assembled to achieve a sealed enclosure. To enhance sealing performance, at least the outer surface of the installation base 3 can be fabricated from an elastic material. This allows for an elastic sealing flange-like connection between the installation base 3 and the housing 2, ensuring leak-proof integrity at their interface and preventing leakage of the aerosol-generating substrate. Furthermore, the installation base 3 may be entirely constructed from an elastic material, such as silicone, and secured via a compression fit to fully seal the liquid storage chamber 21.

To allow external air to enter the vaporizer 6, the installation base 3 is formed with an air intake channel 30 communicating with the air inlet end 61 of the vaporizer 6. The air intake channel 30 allows air to enter, enabling the vaporizer 6 to expel the mixture outward along the path from its air outlet end 62 to the housing's air outlet port 23 after heating the aerosol-generating substrate to produce an aerosol mixed with air.

The control circuit board 4 is connected to the vaporizer 6 and is configured to control the operation of the vaporizer 6, through regulation of the electrified state of the vaporizer 6.

Referring to FIGS. 6-9, to adjust the air intake volume and the operating power of the vaporizer 6 - particularly for synchronously adjusting the air intake volume of the vaporization apparatus 1 and the operating power of the vaporizer 6 to prevent significant deviations between the two - the embodiment of this application further includes an airflow control assembly 5. This airflow control assembly 5 is configured to synchronously regulate both the air intake volume of the vaporization apparatus 1 and the operating power of the vaporizer 6. The air intake of vaporization apparatus 1 affects the flow rate of external air entering the interior of vaporization apparatus 1, which is inversely correlated with the draw resistance of vaporization apparatus 1, meaning that a larger air intake results in lower draw resistance; the operating power corresponds to the heating power output by vaporizer 6, where higher power increases the amount of aerosol generated per unit time by vaporizer 6 heating the aerosol-generating substrate.

Continuing with FIGS. 6-8, to ensure that the amount of aerosol generated matches the volume of external air entering the vaporization apparatus 1, the airflow control assembly 5 specifically includes an adjustment member 51 and a power control switch 52. The adjustment member 51 is positioned at the air inlet port 22 and is movably connected to the housing 2. The adjustment member 51 serves to allow user operation for simultaneously adjusting both the air intake volume and the operating power of the vaporizer 6. To achieve air intake adjustment, the adjustment member 51 includes a shielding portion 53, which is arranged to cover the air intake channel 30. The shielding portion 53 is provided with at least one airflow aperture 530 connected to the air inlet port 22. The shielding portion 53 functions to cover the air intake channel 30, enabling adjustment of the air intake volume of the air intake channel 30 through the airflow aperture 530 on the shielding portion 53. Specifically, the adjustment method involves altering the total cross-sectional area through which the airflow aperture 530 communicates with the air intake channel 30. The larger the total cross-sectional area of communication between the airflow aperture 530 and the air intake channel 30, the greater the air intake volume of the air intake channel 30 becomes, resulting in correspondingly reduced draw resistance; conversely, the smaller the total cross-sectional area of communication between the airflow aperture 530 and the air intake channel 30, the smaller the air intake volume of the air intake channel 30 will be, accompanied by proportionally increased draw resistance.

In some optional embodiments, to adjust the total overlapping area between the airflow aperture 530 and the air intake channel 30, the number of airflow apertures 530 is at least two. The adjustment member 51 is movable relative to the housing 2 to vary the number of airflow apertures 530 communicating with the air intake channel 30, thereby altering the total overlapping area between the airflow aperture 530 and the air intake channel 30. In other words, the embodiment of this application may include multiple airflow aperture 530. During adjustment, the total overlapping area can be modified by selectively aligning different quantities of airflow aperture 530 with the air intake channel 30 based on their number, which in turn changes the air intake volume of the air intake channel 30. The shape of each airflow aperture 530 may be circular, square, elliptical, or other forms, as this is not limited in the embodiment of this application. Additionally, the sizes of the airflow aperture 530 may be uniform or non-uniform. When the sizes are inconsistent, the variation in the total overlapping area between the airflow aperture 530 and the air intake channel 30 during adjustment becomes nonlinear.

In some optional embodiments, to adjust the total connected area between the airflow aperture 530 and the air intake channel 30, the number of airflow apertures 530 is one. The adjustment member 51 is movable relative to the housing 2 to alter the connected area of the airflow aperture 530 itself with the air intake channel 30. In this configuration, with only one airflow aperture 530, the air intake volume of the air intake channel 30 can be modified by either partially or fully aligning the aperture 530 with the channel 30. When the airflow aperture 530 is partially connected to the air intake channel 30, the degree of connectivity may vary, enabling finer granularity in airflow regulation through differentiated adjustment levels. The shape of the airflow aperture 530 is not limited in this embodiment and may be circular, rectangular, elliptical, or other geometric forms.

In addition to the shielding portion 53 configured to adjust the air intake volume of the air intake channel 30, the adjustment member 51 further includes a connecting portion 54 connected to a power control switch 52. The power control switch 52 is electrically connected to the control circuit board 4 and used to control the operating power of the vaporizer 6. The power control switch 52 is mounted on the control circuit board 4, and by operating the power control switch 52, the operating power of the vaporizer 6 can be further regulated. Adjusting the operating power of the vaporizer 6 may involve modifying its operating voltage. In this embodiment, the adjustment member 51 integrates both the shielding portion 53 for adjusting the air intake volume of the air intake channel 30 and the connecting portion 54 for linking to the power control switch 52. When the adjustment member 51 moves relative to the housing 2, the shielding portion 53 alters the total connected area between the airflow aperture 530 and the air intake channel 30, thereby changing the air intake volume of the air intake channel 30 and modifying the draw resistance of the vaporization apparatus 1. Simultaneously, the power control switch 52 is actuated by the connecting portion 54 to adjust the operating power of the vaporizer 6. Notably, the adjustment of the total connected area between the airflow aperture 530 and the air intake channel 30 is synchronized with the adjustment of the vaporizer's operating power, meaning their adjustment directions are aligned. When the total connected area between the airflow aperture 530 and the air intake channel 30 decreases, the operating power of the vaporizer 6 also decreases. Conversely, when the total connected area increases, the vaporizer's operating power increases accordingly. This synchronization can prevent the occurrence of a scenario where a small air intake volume of the air intake channel 30, paired with a higher operating power of vaporizer 6, results in the failure to expel aerosol, thereby causing localized overheating issues in vaporizer 6.

During the adjustment process, the adjustment member 51 is capable of moving relative to the housing 2, such that the total connected area between the airflow aperture 530 and the air intake channel 30 is altered, while simultaneously actuating the power control switch 52 to synchronously adjust the operating power of the vaporizer 6. The relative motion between the adjustment member 51 and the housing 2 may include sliding, rotating, and other forms of displacement.

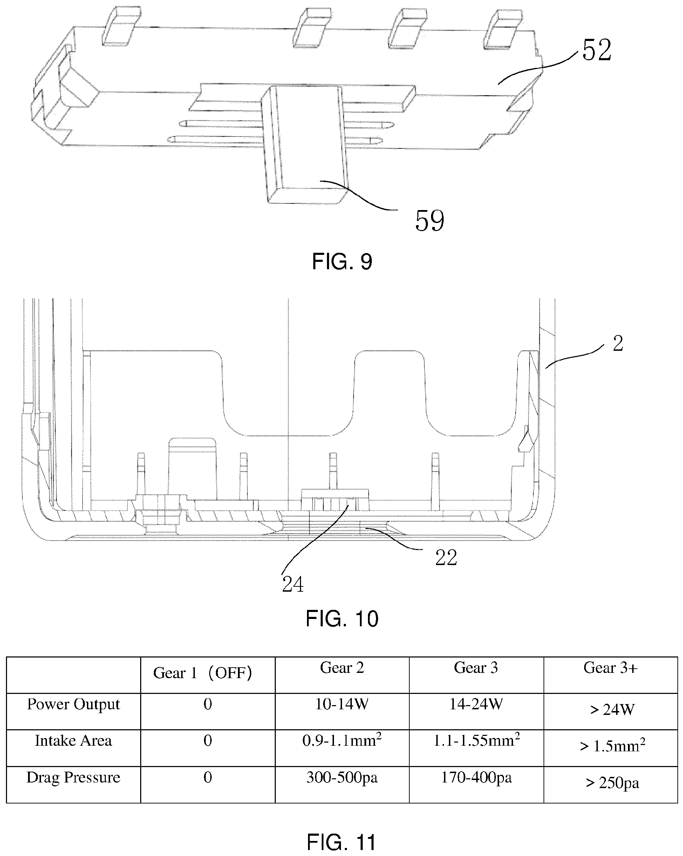

In some optional embodiments, the adjustment process of the adjustment member 51 may be continuous or divided into discrete stages. Referring to Figures 6-8 and 10, elastic protrusions 55 may be disposed on both sides of the adjustment member 51, while the inner side of the housing 2 is provided with at least two sets of the positioning grooves 24 corresponding to the elastic protrusions 55. The elastic protrusions 55 are movably engaged within one set of the positioning grooves 24. The adjustment member 51 can be switched to engage with any set of the positioning grooves 24 under external force. The elastic protrusions 55 directly formed on both sides of the adjustment member 51 allow the adjustment member 51 to be elastically connected to the corresponding positioning grooves 24. The positioning grooves 24, through their interaction with the elastic protrusions 55, provide a positioning function to restrict the movement of the adjustment member 51. Due to the elasticity of the elastic protrusions 55, when external force is applied to the adjustment member 51, the elastic protrusions 55 undergo elastic deformation, contracting inward. This enables the elastic protrusions 55 to disengage from their current positioning grooves 24 and shift between adjacent grooves. After the force is removed, the elastic protrusions 55 return to their original shape and lock into the newly aligned positioning grooves 24. This mechanism allows stage-based adjustment of both the air intake volume of the air intake channel 30 and the operating power of the vaporizer 6. As shown in Figure 11, the correspondence between the vaporizer's operating power, total air intake area of the air intake channel 30, and draw resistance under different adjustment stages is illustrated.

In some optional embodiments, referring to Figures 6-8, to facilitate adjusting the position of the adjustment member 51 relative to the housing 2 and enabling regulation of the air intake volume of the air intake channel 30 and the operating power of the vaporizer 6 via the adjustment member 51, the adjustment member 51 may specifically include a movable base 56 and a sliding button 57 fixed together. The movable base 56 is disposed within an inner accommodating cavity 20 formed by the housing 2, with the shielding portion 53 and the connecting portion 54 both formed on the movable base 56. The sliding button 57 is externally exposed through an air inlet port 22 of the housing 2. Here, the movable base 56 is internally embedded in the housing 2, while the sliding button 57 is externally accessible via the air inlet port 22, allowing users to directly operate the sliding button 57 externally to adjust the air intake volume of the air intake channel 30 and the operating power of the vaporizer 6. For ease of operation, anti-slip protrusions may be added to the surface of the sliding button 57 to increase surface roughness, ensuring more reliable user interaction.

In some optional embodiments, the sliding button 57 and the movable base 56 may be integrally formed as a single piece, or they may be fixedly connected. The sliding button 57 and the movable base 56 can be monolithically manufactured through corresponding production processes, including but not limited to injection molding, compression molding, and similar methods. Alternatively, the sliding button 57 and the movable base 56 may be separately formed components that are later joined into a unified structure via fixed connections. Such fixed connections may employ methods like snap-fit engagement, threaded fastening, interference fit, or adhesive bonding, among others.

In some optional embodiments, to enable the adjustment member 51 to control the power control switch 52, the connecting portion 54 specifically includes a groove 58, and the power control switch 52 includes an actuating lever 59. The actuating lever 59 is inserted into the groove 58, fixedly connecting it to the connecting portion 54. The adjustment member 51 can move relative to the housing 2, driving the actuating lever 59 to shift relative to the control circuit board 4, thereby controlling the operating power of the vaporizer 6. The power control switch 52 incorporates the actuating lever 59. To interface with and control this component, the adjustment member 51 is designed with the groove 58. When the adjustment member 51 is actuated, the actuating lever 59 moves accordingly. During this motion, depending on the circuit configuration of the power control switch 52: if the power control switch 52 already integrates a switching circuit, the actuating lever 59 itself moves relative to the power control switch 52 and the control circuit board 4, enabling the operating power of the vaporizer 6 to be switched. If the switching circuit is formed by the combined structure of the power control switch 52 and the control circuit board 4, the actuating lever 59 drives the power control switch 52 to shift relative to the control circuit board 4, thereby switching the vaporizer's operating power.

In some optional embodiments, continuing to refer to Figures 4 and 5, to facilitate the fixation of components, the control circuit board 4 may be positioned between the airflow control assembly 5 and the installation base 3. The installation base 3 includes an air guide tube 31 communicating with the air intake channel 30. The air guide tube 31 passes through the control circuit board 4 and is fixedly connected to the control circuit board 4. Since the power control switch 52 requires electrical connection to the control circuit board 4, the control circuit board 4 can be arranged adjacent to the airflow control assembly 5 and the installation base 3. Additionally, as the control circuit board 4 typically requires internal wiring within the housing 2, its fixation method must be highly stable. Therefore, the installation base 3 can further be connected to the control circuit board 4. The air guide tube 31, which communicates with the air intake channel 30 on the installation base 3, penetrates and is fixedly connected to the control circuit board 4, thereby ensuring mutual fixation between the control circuit board 4 and the installation base 3.

In some optional embodiments, continuing to refer to Figures 6-8, to enhance the sealing between the shielding portion 53 and the air intake channel 30-ensuring that the air intake channel 30 communicates with the external environment solely through the airflow aperture 530 and preventing leakage at the junction between the shielding portion 53 and the air intake channel 30-the end of the air guide tube 31 is provided with an elastic sealing flange 32. The elastic sealing flange 32 elastically contacts the shielding portion 53 to seal the air intake channel 30. By leveraging the elasticity of the elastic sealing flange 32 at the end of the air guide tube 31, a tightly sealed connection is achieved between the end of the air guide tube 31 and the shielding portion 53, ensuring robust sealing performance.

The embodiments of the present application provide a vaporization apparatus 1. Due to the connection relationships between the adjustment member 51, the air intake channel 30, and the power control switch 52, adjusting the position of the adjustment member 51 relative to the housing 2 enables simultaneous regulation of both the airflow volume and the operating power of the vaporizer 6 through a single operation of the adjustment member 51. This effectively avoids mismatches between airflow volume and operating power, extends the service life of the vaporization device, and enhances user experience.

The embodiments of the present application further provide a vaporization device. As shown in Figures 1 and 2, the vaporization device includes a power supply assembly 7 and the aforementioned vaporization apparatus 1.

The power supply assembly 7 is fixedly installed within the inner accommodating cavity 20 of the housing 2. The power supply assembly 7 is configured to supply electrical power to the vaporizer 6. Specifically, the power supply assembly 7 may be connected to the control circuit board 4, while the power control switch 52, which is electrically connected to the control circuit board 4, regulates the operating power of the vaporizer 6. This vaporization device may be designed as a disposable product or a cartridge-replaceable product. For disposable vaporization devices, the vaporizer 6, power supply assembly 7, and housing 2 are permanently connected. In cartridge-replaceable vaporization devices, the vaporizer 6 and power supply assembly 7 are detachably connected, allowing replacement of either component based on usage requirements.

The above explanation utilizes specific examples to illustrate the utility patent, which are intended solely to aid in understanding the technical solution and should not be construed as limiting the scope of the utility patent. For technical personnel in the field relevant to this utility patent, various simple deductions, modifications, or substitutions may be derived based on the core principles of the utility patent while remaining within the framework of its technical spirit. Any such adaptations shall fall within the protection scope defined by the claims of this utility patent.

## Claims

1. A vaporization apparatus, comprising:
a housing, wherein the housing forms an inner accommodating cavity, the inner accommodating cavity including a liquid storage chamber configured to store an aerosol-generating substrate; the housing further defining an air inlet port and an air outlet port in communication with external environment;
a vaporizer, wherein the vaporizer is disposed within the liquid storage chamber; the vaporizer comprising an air inlet end and an air outlet end arranged oppositely, wherein the air outlet end communicates with the air outlet port;
an installation base, wherein the installation base is fixedly assembled to an end of the liquid storage chamber distal from the air outlet port, sealing the liquid storage chamber; the installation base is formed with an air intake channel communicating with the air inlet end of the vaporizer;
a control circuit board, wherein the control circuit board is electrically connected to the vaporizer and is configured to control operation of the vaporizer;
an airflow control assembly, wherein the airflow control assembly comprises an adjustment member and a power control switch, the adjustment member is disposed at the air inlet port and is movably connected to the housing; the adjustment member includes a shielding portion covering the air intake channel, the shielding portion is provided with at least one airflow aperture communicating with the air inlet port; the adjustment member further includes a connecting portion linked to the power control switch; the power control switch is electrically connected to the control circuit board to regulate an operational power of the vaporizer; the adjustment member is movable relative to the housing to alter a total effective area of the at least one airflow aperture communicating with the air intake channel, while simultaneously drives the power control switch to synchronously adjust the operational power of the vaporizer.

2. The vaporization apparatus according to claim 1, wherein
a number of airflow apertures is at least two; the adjustment member is movable relative to the housing to vary the number of airflow apertures communicating with the air intake channel, thereby changing the total effective area.

3. The vaporization apparatus according to claim 1, wherein
a number of airflow apertures is one; the adjustment member is movable relative to the housing to adjust the total effective area of the at least one airflow aperture communicating with the air intake channel.

4. The vaporization apparatus according to any one of claims 1-3, wherein
two sides of the adjustment member are provided with elastic protrusions, and an inner wall of the housing is provided with at least two sets of positioning grooves matching the elastic protrusions; the elastic protrusions are movably engaged with one set of the positioning grooves, and the adjustment member is switchable under external force to engage with any set of the positioning grooves.

5. The vaporization apparatus according to any one of claims 1-3, wherein
the adjustment member comprises a movable base and a sliding button fixedly interconnected; the movable base is disposed within the inner accommodating cavity of the housing, with the shielding portion and the connecting portion both formed on the movable base; the sliding button is exposed outside the housing through the air inlet port.

6. The vaporization apparatus according to claim 5, wherein
the sliding button and the movable base are integrally formed; or, the sliding button and the movable base are fixedly connected.

7. The vaporization apparatus according to any one of claims 1-3, wherein
the connecting portion comprises a groove, and the power control switch includes an actuating lever inserted into the groove to fixedly couple the actuating lever to the connecting portion; a movement of the adjustment member relative to the housing drives the actuating lever to move relative to the control circuit board, thereby controlling the operational power of the vaporizer.

8. The vaporization apparatus according to any one of claims 1-3, wherein
the control circuit board is disposed between the airflow control assembly and the installation base; the installation base includes an air guide tube communicating with the air intake channel, the air guide tube penetrates the control circuit board and is fixedly connected to the control circuit board.

9. The vaporization apparatus according to claim 8, wherein
an end of the air guide tube is provided with an elastic sealing flange elastically contacting the shielding portion to seal the air intake channel.

10. A vaporization device, comprising a power supply assembly and the vaporization apparatus according to any one of claims 1-9; the power supply assembly is fixedly disposed within the inner accommodating cavity of the housing and is configured to supply power to the vaporizer.
